# EUROPEAN PATENT APPLICATION

(11) **EP 3 115 001 A1**
(43) Date of publication of application: **11.01.2017**
(21) Application number: 15758233.9
(22) Date of filing: 10.02.2015
(51) Int. Cl.: A61B 17/00, A61B 1/00

(54) **CAP FOR MEDICAL DEVICE**

(30) Priority: 04.03.2014 JP 2014041112
(71) Applicant: Olympus Corporation, Tokyo 192-8507 (JP)
(72) Inventor: HYODO, Ryoji, Tokyo 151-0072 (JP); KISHI, Kosuke, Tokyo 151-0072 (JP)
(74) Representative: Schicker, Silvia
(86) International application number: PCT/JP2015/053577
(87) International publication number: WO 2015/133230

(57) **Abstract**

The invention has for its object to provide a cap for medical equipment that is capable of improving on the operability of a treatment part while acting as a cover for decreasing stains.

A cap 10 for medical equipment includes a base member 11 attached to a distal end of medical equipment 1 and portions 12, 13, 14 and 15 extending out of the base member 11, wherein the portions 12, 13, 14 and 15 are put into passive operation.

## Description

### Technical Field

The present invention relates to a cap for medical equipment that is used with medical equipment capable of being inserted in the body cavity of a patient during surgical operation or the like for the purpose of viewing, treatments and so on.

### Background Art

There has been extensive use of medical equipment including an elongate insert part in which the insert part is inserted in the body cavity of a patient and the distal end of the insert part or the like is hauled by means of a cable or the like for the purpose of viewing, and applying treatments to, organs in the body cavity. It is highly likely that the distal end of this medical equipment may be stained thanks to the fact that it is used for viewing, and applying treatments to, organs in the body cavity. For this reason, it has been proposed to provide the distal end of medical equipment with a cap.

For a general method for treatment of a digestive tract lesion, there is endoscopic mucosal resection (EMR) wherein the lesion is endoscopically resected, and endoscopic submucosal dissection (hereinafter ESD for short) in particular is known as endoscopic treatment that provides an unerring method in which after the incision of a mucosa around the lesion, a submucosa is peeled off for dissection of the lesion so that the lesion can be dissected in the lump.

At the time of such ESD, a physiological saline solution or the like is injected into a normal mucosa around a lesion by way of an injection needle to dissect the lesion from the normal mucosa by use of a high-frequency incision tool such as a high-frequency knife or snare while the lesion is floated up. In this case, the lesion is lifted up to a position enough to take hold of a portion of a boundary to be dissected between the lesion and the normal mucosa or when the lesion has a flat shape, a portion to be dissected is created. To this end, a transparent cap attached to the distal end of an endoscope is slipped under the mucosa to get on with the incision of a submucosa by means of a high-frequency incising tool while the mucosa is lifted up.

Since the distal end of the transparent cap has a distal end diameter greater than the distal end diameter of the endoscope, however, it is still hard to slip the cap under a fine incised wound thereby creating a new wound, and even with the cap slipped inside, the mucosa slips off the cap upon endoscopic operation for incision and peeling. In that case it is required to again slip the cap inside.

Patent Literature 1 discloses a cap whose diametrical size is variable as by an operating wire in such a way as to be smaller than the outer size of the distal end surface of the insert part of an endoscope so as to make it easier and smoother for the cap to be slipped under.

### Citation List

### Patent Literature

Patent Literature 1: JP(A) 2012-239833

### Summary of Invention

### Technical Problem

With the technology set forth in Patent Literature 1, however, the cap extends from the distal end all over the endoscope. Therefore, when an affected site is treated by a jointed treatment part, the cap interferes with the joints upon movement within the cap, resulting in the need for increasing the length of the treatment part in such a way as to extend out of the end of the cap. Even when the treatment part is made long, it is difficult to treat the affected site in the case where it is present around the cap. The need for using a wire to drive the cap also results in an increase in the diameter of the endoscope or cap, which leads to a complicated mechanism involved.

With the aforesaid problem in mind, the present invention has for its object to provide a cap for medical equipment that improves on the operability of a treatment part while, at the same time, acts as a cover capable of decreasing the deposition of stains.

### Solution to Problem

According to one embodiment, a cap for medical equipment includes:
a base member attached to a distal end of medical equipment, and
an extension assembly that extends out of the base member, wherein the extension assembly is put into passive operation.

In the cap for medical equipment according to one embodiment, the extension assembly is put into operation as a treatment part used upon treatment of an affected site by the medical equipment is put into operation.

In the cap for medical equipment according to one embodiment, the extension assembly includes two lateral portions supported on the base member, and a front portion coupled to the two lateral portions.

In the cap for medical equipment according to one embodiment, the front portion is provided with a surgical opening through which the treatment part can be inserted.

The cap for medical equipment according to one embodiment further includes a plurality of the surgical openings.

In the cap for medical equipment according to one embodiment, at least one of the surgical openings has an insertion length different from that of the other surgical opening(s).

In the cap for medical equipment according to one embodiment, the front portion is transparent or semitransparent.

In the cap for medical equipment according to one embodiment, the lateral portions each include an arm portion and a shaft portion for swingably connecting the arm portion to the base member.

In the cap for medical equipment according to one embodiment, the arm portion includes a link member having a proximal end and a distal end, and a coupling member for swingably connecting the base member and the shaft member to a shaft at the proximal end and for rotatable attachment of the front portion at the distal end.

In the cap for medical equipment according to one embodiment, the arm portion can be retained at a plurality of angles relative to the base member.

In the cap for medical equipment according to one embodiment, the base member is provided with a stopper in such a way as to restrict an angle of swinging of the arm member.

In the cap for medical equipment according to one embodiment, the arm portion is capable of movement relative to the shaft member.

In the cap for medical equipment according to one embodiment, the arm portion includes an opening formed as a slot for movably supporting the shaft member.

The cap for medical equipment according to one embodiment includes a treating mode capable of putting the extension assembly into passive operation, and a moving mode of moving the arm portion of the extension assembly on a base member side relative to the shaft member and retaining there.

In the cap for medical equipment according to one embodiment, the lateral portion includes flexible arms one of which is supported on the base member and the other of which is coupled to the front portion.

### Advantageous Effects of Invention

The cap for medical equipment according to one embodiment makes it possible to improve on the operability of a treatment part while, at the same time, acts as a cover for reducing stains.

### Brief Description of Drawings

Fig. 1 is a schematic view of the cap for medical equipment according to the first embodiment.
Fig. 2 is a schematic view of one exemplary operating state of the cap for medical equipment according to the first embodiment.
Fig. 3 is a schematic view of one exemplary state where the medical equipment is allowed to carry out treatment using the cap for medical equipment according to the first embodiment.
Fig. 4 is a schematic view of the cap for medical equipment according to the second embodiment.
Fig. 5 is a schematic view of one exemplary state of the cap for medical equipment according to the second embodiment, to which the treatment tool is attached.
Fig. 6 is a schematic view of an initial state where the affected site is treated by the medical equipment using the cap for medical equipment according to the second embodiment.
Fig. 7 is a schematic view of a state of the medical equipment using the cap for medical equipment according to the second embodiment immediately before the surgical distal-end portion is slipped deep under the affected site.
Fig. 8 is illustrative of the first operation for pulling the surgical distal-end portion of the medical equipment out of the cap for medical equipment according to the second embodiment.
Fig. 9 is illustrative of the second operation for pulling the surgical distal-end portion of the medical equipment out of the cap for medical equipment according to the second embodiment.
Fig. 10 is illustrative of the third operation for pulling the surgical distal-end portion of the medical equipment out of the cap for medical equipment according to the second embodiment.
Fig. 11 is a schematic view of one exemplary state where the surgical distal-end portion of the medical equipment using the cap for medical equipment according to the second embodiment is slipped deep under the affected site.
Fig. 12 is a schematic view of the cap for medical equipment according to the third embodiment.
Fig. 13 is a schematic view of the cap for medical equipment according to the third embodiment near to the shaft member.
Fig. 14 is a schematic view of one exemplary state where the treatment part turns downward with respect to the cap for medical equipment according to the fourth embodiment.
Fig. 15 is a schematic view of one exemplary state where the treatment part turns upward with respect to the cap for medical equipment according to the fourth embodiment.
Fig. 16 is a view of Figs. 14 and 15 as taken on XVI-XVI section.
Fig. 17 is a schematic view of the cap for medical equipment according to the fifth embodiment.
Fig. 18 is a schematic view of the cap for medical equipment according to the sixth embodiment.
Fig. 19 is a schematic view of the cap for medical equipment according to the seventh embodiment.
Fig. 20 is illustrative of one example of the surgical opening in the cap for medical equipment according to one embodiment.
Fig. 21 shows one exemplary shape of the front portion of the blade in the cap for medical equipment according to one embodiment.
Fig. 22 is illustrative of the surgical system to which the medical equipment incorporating the cap for medical equipment according to one embodiment is applied.
Fig. 23 is illustrative in system architecture of the surgical system to which the medical equipment incorporating the cap for medical equipment according to one embodiment is applied.

### Description of Embodiments

Embodiments are now explained.

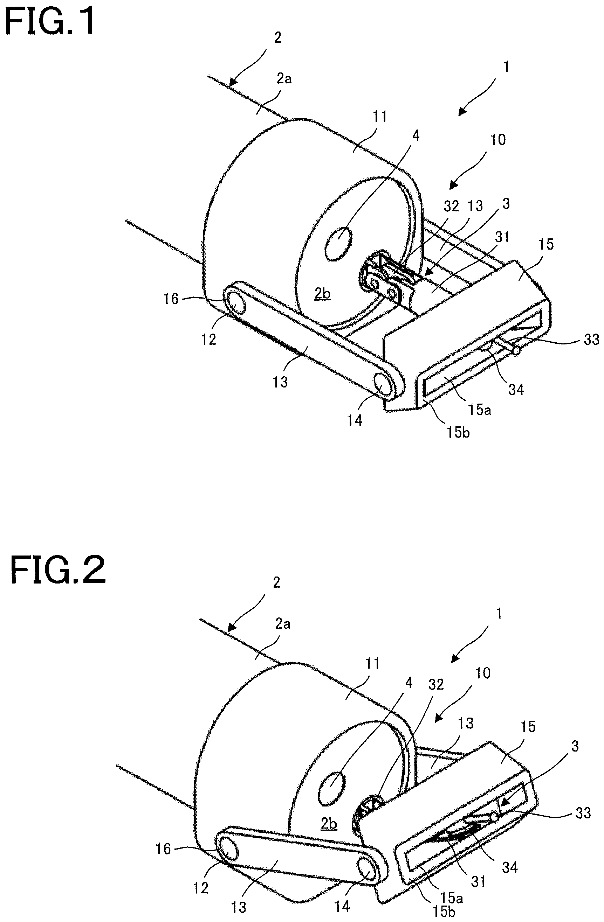

Fig. 1 is a schematic view of the cap 10 for medical equipment according to the first embodiment.

The cap 10 for medical equipment according to the first embodiment includes a base member 11 attached to a distal end portion 2a of an insert part 2 of a medical equipment 1, a shaft member 12 attached to the base member 11, a link member 13 swingably supported on the shaft member 12, a coupling member 14 attached to the link member 13, and a blade 15 rotatably supported on the coupling member 14. The shaft member 12, link member 13, coupling member 14 and blade 15 define together an extension assembly; the shaft member 12, link member 13 and coupling member 14 define together a lateral portion; the blade 15 defines a front portion; and the link member 13 and coupling member 14 define together an arm portion.

The base member 11 is detachably mounted on the outer circumference of the distal end portion 2a of the medical equipment 1. The base member 11 is provided with two shaft members 12. Extending from the base member 11 are two link members 13 each one of which has a proximal end swingably supported by the shaft member 12. Each shaft member 12 is inserted into a mount opening 16 formed in the associated link member 13. The distal ends of two such link members 13 are provided with the coupling members 14, respectively, and both ends of the blade 15 are rotatably supported by the two coupling members 14. The blade 15 is provided with a surgical opening 15a through which a treatment part 3 can be inserted. Note here that with the shaft members 12 mounted on the link members 13 and the mount openings 16 formed in the base member 11, the shaft members 12 may be inserted into the mount opening 16 to support the link members 13 in a swingable manner.

As shown in Fig. 1, the treatment part 3 inserted through the medical equipment 1 for use includes a main portion 31, a first joint portion 32 that bendably supports the main portion 31, a surgical distal-end portion 33 attached to the distal end of the main portion 31, and a second joint portion 34 that makes the surgical distal-end portion 33 bendable in a direction intersecting the bending direction of the first joint portion 32, and the treatment part 3 is mounted in such a way as to be rotatable relative to an axis in a linear state. The distal portion 2a is provided at its distal-end surface 2b with an endoscope 4. Note here that the cap 10 for medical equipment is preferably formed of a transparent or semitransparent material so as to allow the user to have a good view of images taken through the endoscope 4.

Fig. 2 is a schematic view of one exemplary operating state of the cap 10 for medical equipment according to the first embodiment.

As the cap 10 for medical equipment is attached to the distal-end portion 2a of the medical equipment 1, it enables the surgical distal-end portion 33 of the linearly extending treatment part 3 to pass through the surgical opening 15a. As the first joint portion 32 of the treatment part 3 is bent from a state where the treatment distal-end portion 33 passes through the surgical opening 15a, it causes the blade 15 of the cap 10 for medical equipment to be pushed by the surgical distal-end portion 33, as shown Fig. 2, resulting in a swinging movement of the link members 13 with the shaft members 12 as center. To put it another way, the shaft members 12, link members 13, coupling members 14 and blade 15 defining together the extension assembly are put into passive operation.

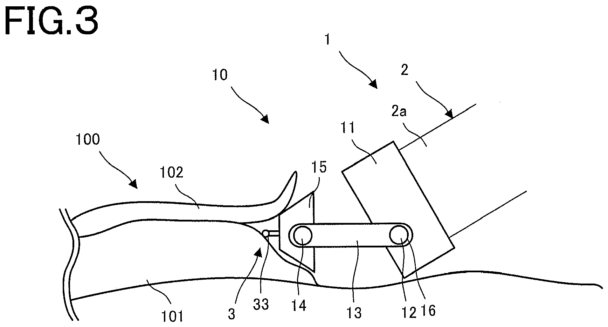

Fig. 3 is a schematic view of one exemplary state where the medical equipment 1 is allowed to apply treatment to an affected site 100 using the cap 10 for medical equipment according to the first embodiment.

When the medical equipment 1 is used to apply treatment to the affected site 100 in the case of ESD or the like as shown in Fig. 3, it is required for the medical equipment 1 to make use of the surgical distal-end portion 33 of the treatment part 3 to cut open between a mucosa 102 and a submucosa 101. At this time the mucosa 102 gets in the way. With the cap 10 for medical equipment according to the first embodiment, therefore, the mucosa 102 is forced up by the blade 15 to slip the treatment part 3 under the submucosa 101. Note here that the taper of the blade 15 allows the treatment part 3 to be smoothly slipped under.

With the cap 10 for medical equipment according to the first embodiment, therefore, the mucosa 102 is unlikely to slip down so that the endoscope 4 shown in Fig. 1 can present clear-cut images of treatment of the affected site 100 by the treatment part 3. The cap 10 for medical equipment according to the first embodiment is driven in harmony with the movement of the treatment part 3 so that the affected site 100 can be treated with no interference between the treatment part 3 and the cap 10.

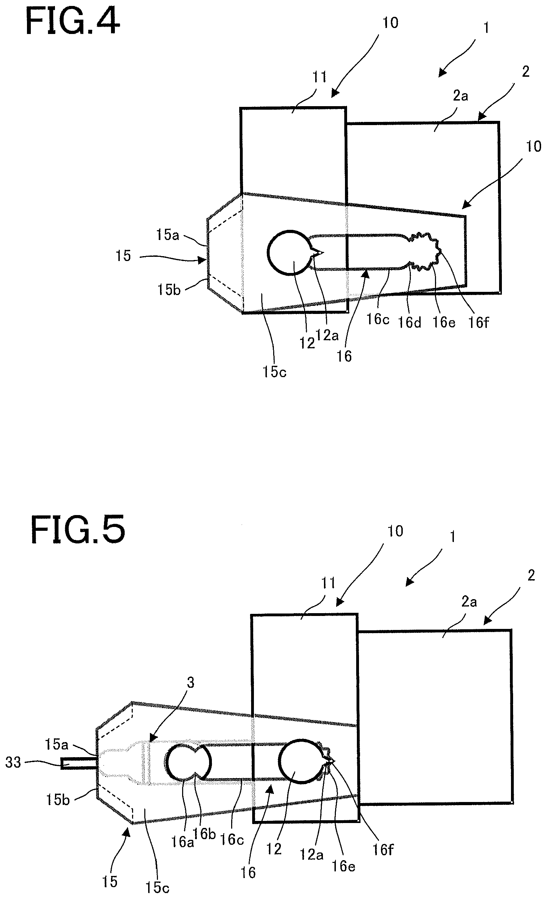

Fig. 4 is a schematic view of the cap 10 for medical equipment according to the second embodiment, and Fig. 5 is a schematic view of the cap 10 for medical equipment according to the second embodiment, on which the treatment part 3 is mounted.

The cap 10 for medical equipment according to the second embodiment includes a base member 11 attached to a distal-end portion 2a of a medical equipment 1, a shaft member 12 attached to the base member 11, and a blade 15 that is swingably supported on the shaft member 12. The shaft member 12 and blade 15 define together an extension assembly.

In the cap 10 for medical equipment according to the second embodiment, the shaft member 12 attached to the base member 11 is provided with an engaging protrusion 12a that protrudes from its outer circumference in a diametrical direction. The blade 15 includes a front portion 15b opposing normally to the distal-end surface 2b of the distal-end portion 2a and two side portions 15c that extend from both ends of the front portion 15b toward the shaft member 12. The shaft member 12 and side portions 15c define together a lateral portion while the side portions 15c define an arm portion.

As is the case with the first embodiment, the front portion 15b is provided with a surgical opening 15a through which the distal-end portion 33 of the treatment part 3 can be inserted. Each side portion 15c is provided with a mount opening 16 that is mounted on the shaft member 12. The mount opening 16 includes a succession of a first support portion 16a, a first retaining portion 16b, a guide portion 16c, a second retaining portion 16d and a second support portion 16e, the mount opening 16 has a slot form. The second support portion 16e is provided with a plurality of engaging recesses 16f.

The first support portion 16a retains the shaft member 12 at a time when the treatment part 3 is not installed as shown in Fig. 4, and has a substantially circular shape. The first retaining portion 16b retains the shaft member 12 such that it does not move to the guide portion 16c at a time when the shaft member 12 is retained on the first support portion 16a as shown in Fig. 4. The guide portion 16c guides the shaft member 12 from the first support portion 16a to the second support portion 16e upon a transition from the state shown in Fig. 4 to the state shown in Fig. 5. The first retaining portion 16b retains the shaft member 12 such that it does not move to the guide portion 16c at a time when the shaft member 12 is retained on the second support portion 16e, as shown in Fig. 5. The second support portion 16e retains the shaft member 12 at a time when the treatment part 3 is installed, as shown in Fig. 5, and has a substantially circular shape.

Until the medical equipment 1 gains access to the affected site, the shaft member 12 is retained by the first support portion 16a and the first retaining portion 16b; the blade 15 is unlikely to stagger, block off the field of vision of the medical equipment 1 or hinder the movement of the medical equipment 1. Especially when the medical equipment 1 is an endoscope, the mode (hereinafter called the moving mode) without any detriment to the insertion of the endoscope down to the affected site is of great importance.

While the shaft member 12 is retained on the second support portion 16e, a plurality of engaging recesses 16f are in engagement with the engaging protrusion 12a provided on the shaft member 12 to retain the blade 15 in place, as shown in Fig. 5. Depending on the positions of the engaging recesses 16f in engagement with the engaging protrusion 12a, the blade 15 is capable of being retained at a plurality of angles. Note here that the protrusion may be formed on the base member 11 while the blade 15 may be provided with an opening through which the protrusion can be inserted.

To make a transition from the state of Fig. 4 that defines the mode of moving the medical equipment 1 to the affected site to the state of Fig. 5, the treatment part 3 mounted on the medical equipment 1 is pushed out of it to move the shaft member 12 to the second support portion 16e of the blade 15 in the treating mode.

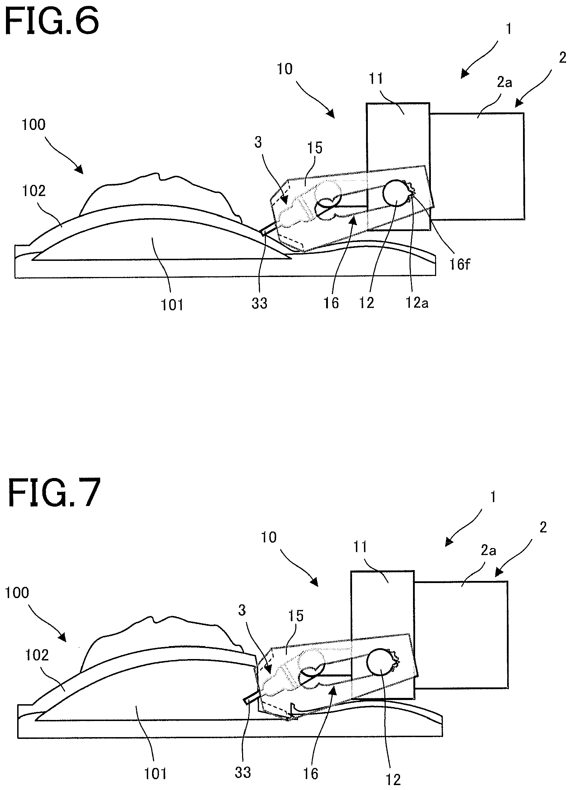

Figs. 6 to 11 are schematic views of exemplary states where the affected site 100 is treated by the medical equipment 1 using the cap 10 for medical equipment according to the second embodiment.

Fig. 6 is a schematic view of an initial state where the affected site 100 is treated by the medical equipment 1 using the cap 10 for medical equipment according to the second embodiment.

When the affected site 100 is treated by the medical equipment 1, it is required for the surgical distal-end portion 33 of the treatment part 3 to abut upon the affected site 100, as shown in Fig. 6. At this time, when the affected site 100 cannot be treated unless the treatment part 3 is bent, it is preferred that the engaging protrusion 12a formed on the shaft member 12 is brought in engagement with a given position of the engaging recesses 16f to retain the blade 15 at a given angle. Retaining the blade 15 at a given angle makes it possible to use the treatment part 3 in a stabilized state.

Fig. 7 is a schematic view of a state of the medical equipment 1 using the cap 10 for medical equipment according to the second embodiment immediately before the surgical distal-end portion 33 is slipped deep under the affected site 100.

When a region under the protrusion is treated, the cap is taken out of the blade to treat the region below the cap. In this case, the blade is retained at a given angle to carry out treatment without causing the mucosa to slip down on the region to be incised open.

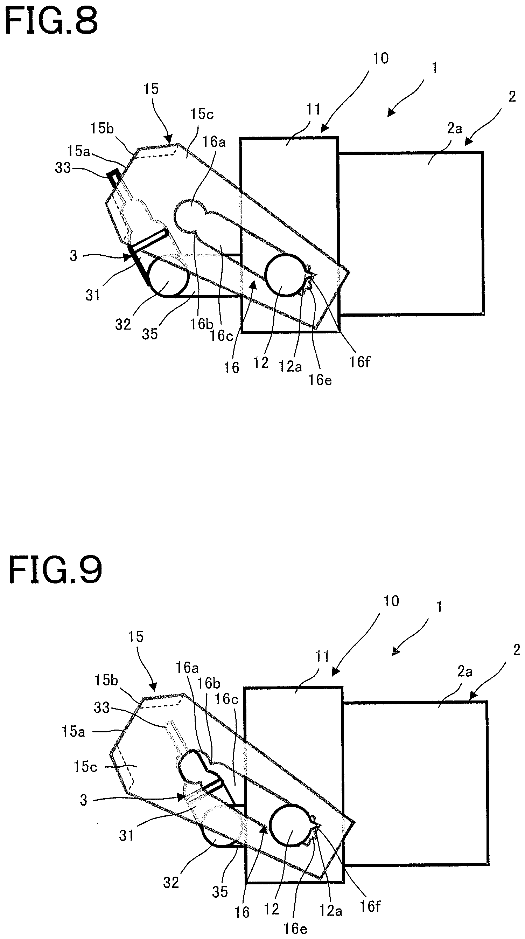

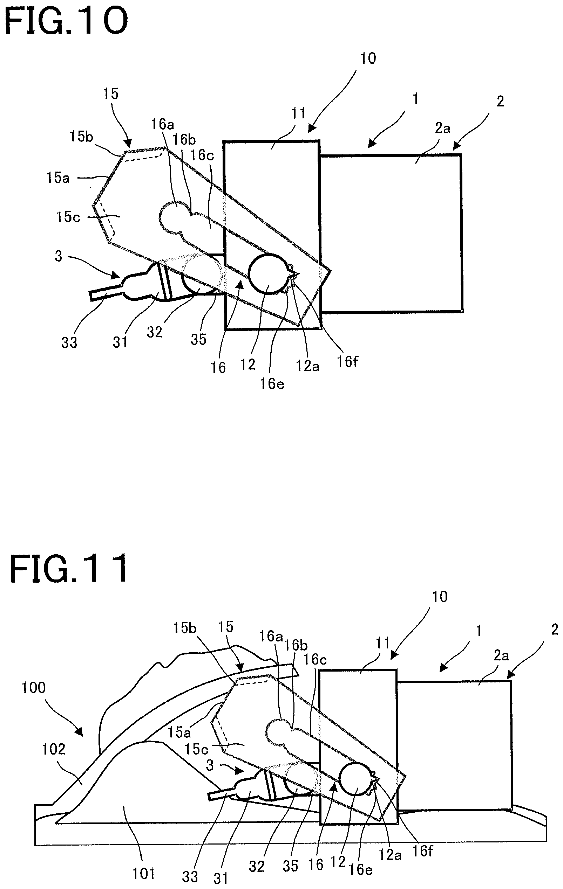

Fig. 8 is illustrative of the first operation for pulling the surgical distal-end portion 33 of the medical equipment 1 out of the cap 10 for medical equipment according to the second embodiment; Fig. 9 is illustrative of the second operation for pulling the surgical distal-end portion 33 of the medical equipment 1 out of the cap 10 for medical equipment according to the second embodiment; and Fig. 10 is illustrative of the third operation for pulling the surgical distal-end portion 33 of the medical equipment 1 out of the cap 10 for medical equipment according to the second embodiment.

When the submucosa 101 of the affected site shown in Fig. 7 is treated, the cap 10 for medical equipment is driven in harmony with the movement of the treatment part 3 so that the affected site 100 can be treated with no interference between the treatment part 3 and the cap 10, as is the case with the first embodiment shown in Fig. 3.

First, the extension portion 35 of the treatment part 3 is extended outwardly from the distal-end portion 2a as is the case with the first operation shown in Fig. 8 while, at the same time, the first joint portion 32 is bent to let the surgical opening 15a in the blade 15 retained at a given angle oppose to the position of the surgical distal-end portion 33.

Then, as is the case with the second operation shown in Fig. 9, a part of the extension portion 35 of the treatment part 3 is housed in the distal-end portion 2a and the surgical distal-end portion 33 is pulled from within the surgical opening 15a in the blade 15.

Then, as is the case with the third operation shown in Fig. 10, while the blade 15 remains retained at a given angle, the first joint portion 32 of the treatment part 3 is bent to a position capable of treatment.

Fig. 11 is a schematic view of one exemplary state where the surgical distal-end portion 33 of the medical equipment 1 using the cap 10 for medical equipment according to the second embodiment is slipped deep under the affected site 100.

Referring here to the cap 10 for medical equipment according to the second embodiment, the blade 15 is slipped under the mucosa 102 of the affected site 100 from below to push up the mucosa 102 so that the treatment part 3 is slipped down the submucosa 101.

With the cap 10 for medical equipment according to the second embodiment, therefore, images of the affected site 100 under treatment by the treatment part 3 can be clearly shown by the endoscope 4 depicted in Fig. 1 because the mucosa 102 is unlikely to slip down. With the cap 10 for medical equipment according to the second embodiment, the affected site 100 can be treated with no interference between the treatment part 3 and the cap 10 because the cap 10 is driven in harmony with the movement of the treatment part 3. Further with the cap 10 for medical equipment according to the second embodiment, it is possible to improve on the operability of the treatment part 3 because the blade 15 is retained at a given angle relative to the base member 11 without imposing restrictions on the movement of the treatment part 3. Note here that the engaging protrusion 12a and engaging recesses 16 define together a stopper.

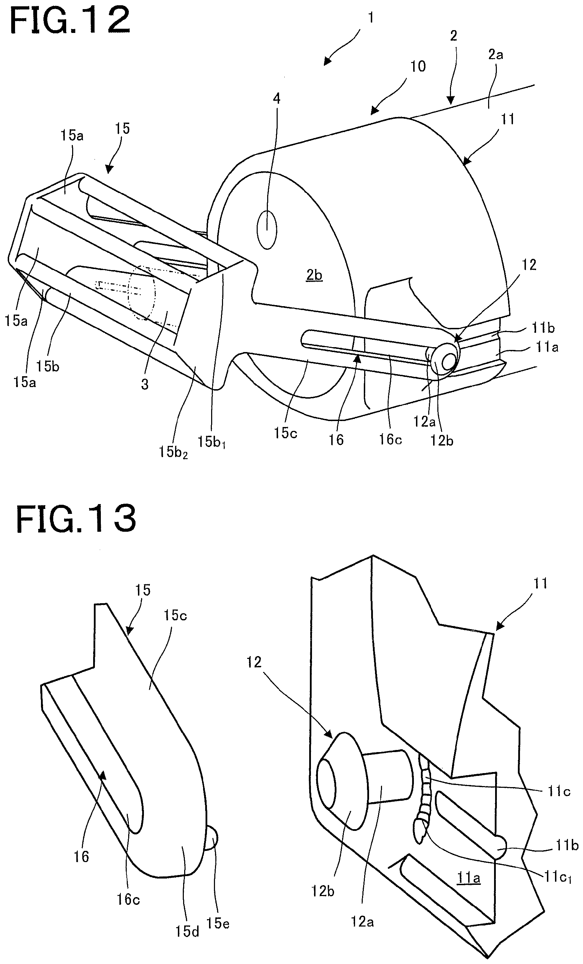

Fig. 12 is a schematic view of the cap 10 for medical equipment according to the third embodiment, and Fig. 13 is a schematic view of the cap 10 for medical equipment according to the third embodiment near to the shaft member 12.

As in the second embodiment, the cap 10 for medical equipment according to the third embodiment has a structure capable of retaining the blade 15 at a plurality of angles.

The cap 10 for medical equipment according to the third embodiment includes a base member 11 attached to a distal-end portion 2a of a medical equipment 1, a shaft member 12 attached to the base member 11, and a blade 15 swingably supported on the shaft member 12. The shaft member 12 and blade 15 define together an extension assembly, and the shaft member 12 and side portion 15c define together a lateral portion while the side portion 15c defines an arm portion.

In the cap 10 for medical equipment according to the third embodiment, a part of the base member 11 including a position of supporting the shaft member 12 is provided with a planar step 11a that is provided with a groove 11b and an engaging recess 11c. The groove 11b has a linear shape in a direction of extension of the insert part 2. The engaging recess 11c is positioned between the shaft member 12 and the groove 11b, and has an arc shape with the shaft member 12 as center. The engaging recess 11c includes a plurality of serrations and there is a crest 11c₁ between adjoining serrations provided to delimit the serrations.

The shaft member 12 includes a shaft portion 12a and a diameter-increased portion 12b formed at the distal end of the shaft portion 12a with a diameter larger than that of the shaft portion 12a, and the diameter-increased portion 12b is tapered off.

The blade 15 includes a front portion 15b normally opposite to the distal-end surface 2b of the distal-end portion 2a, and two side portions 15c that extend from both ends of the front portion 15b toward the shaft member 12. The shaft member 12 and side portions 15c define together a lateral portion, and the side portions 15c define together an arm portion.

On both sides of the direction of movement of the blade 15, the front portion 15b is provided with a first slope 15b₁ and a second slope 15b₂, each one of which is tapered off, and with surgical opening 15a through which the distal-end portion 33 of the treatment part 3 shown in Fig. 2 can be inserted as in the second embodiment. The first and second slopes 15b₁ and 15b₂ are each provided with a surgical opening 15a as well. Note here that either one or both of the first and second slopes 15b₁ and 15b₂ may be provided, and the surgical opening 15a may or may not be formed in either one of the first and second slopes 15b₁ and 15b₂. Alternatively, the surgical openings 15a in the front portion 15b and the first and second slopes 15b₁ and 15b₂ may be each provided in the form of a plurality of narrow slots. Each side portion 15c is formed with a mount opening 16 to be attached to the shaft member 12. The mount opening 16 is provided with a guide portion 16c in the form of a slot as in the second embodiment. Between the mount opening 16 in the side portion 15c and the distal-end portion 15d there is an engaging protrusion 15e provided in such a way as to protrude inwardly.

The cap 10 for medical equipment having such a structure according to the third embodiment is assembled by attaching the base member 11 to the distal-end portion 2a of the insert part 2 and inserting the shaft member 12 including the integrated shaft portion 12a and diameter-increased portion 12b into the mount opening 16 in the blade 15. The diameter of the diameter-increased portion 12b of the shaft member 12 is larger than the short width of the mount opening 16 and the diameter of the shaft portion 12a is substantially identical with the short width of the mount opening 16. Thus, when the shaft member 12 has already been attached to the base member 11, the blade 15 is fitted into the mount opening 16 such that the mount opening 16 goes over the diameter-increased portion 12b in a tapered shape. Note here that how to attach the shaft member 12 to the mount opening 16 is not limited to what is described above. For instance, the diameter-increased portion 12b may be provided as a separate one and the shaft portion 12a may be inserted into the mount opening 16, after which the diameter-increased portion 12b is fixed as by fitting it over the outer circumference of the tapered part of the shaft portion 12a.

As the blade 15 is attached to the shaft member 12, it causes the engaging protrusion 15e to be fitted into the groove 11b or engaging recess 11c. When the engaging protrusion 15e is fitted into the groove 11b, the blade 15 is movable to the position of the moving mode in which it is stored in the side of the insert part 2 relative to the cap 10 for medical equipment as well as to the treating mode in which it is stored away from the insert part 2.

Upon delivery of the medical equipment 1 to the affected site, the shaft member 12 is retained by the groove 11b ensuring that the blade 15 does not stagger to prevent it from blocking off the field of vision of the medical equipment 1 and disturbing the movement of the medical equipment 1. Especially when the endoscope 4 is used as the medical equipment 1, the present mode without detrimental to deliverability to the affected site (herein termed the moving mode) is of great importance.

As the engaging protrusion 15e of the blade 15 engages the engaging recess 11c and is retained on the crest 11c₁, it causes the blade 15 to be retained at a given angle relative to the base member 11. Depending on the position of the engaging recess 11c in engagement with the engaging protrusion 15e, the blade 15 may be retained at a plurality of angles. Note here that the engaging protrusion 15e may be formed on the base member 12 and the engaging recess may be formed in the blade 15.

By attaching the treatment part 3 to the medical equipment 1 and pushing the treatment part 3 out of the medical equipment 1, the moving mode for delivery of the medical equipment 1 to the affected site may make a shift to the treating mode in which the engaging protrusion 15e of the blade 15 moves from the groove 11b to the engaging recess 11c.

With the cap 10 for medical equipment according to the third embodiment, therefore, it is unlikely that the mucosa 102 slips down, as is the case with the cap 10 for medical equipment according to the second embodiment shown in Fig. 10, ensuring that images of the affected site 100 under treatment by the treatment part 3 can be clearly shown by the endoscope 4. The third embodiment also makes the cap 10 for medical equipment, because of being driven in harmony with the movement of the treatment part 3, unlikely to interfere with the treatment part 3, ensuring that the affected site 100 can be treated. Further, the cap 10 for medical equipment according to the third embodiment ensures that the blade 15 is retained at a given angle relative to the base member 11, preventing the blade 15 from imposing limitation on the treatment part 3 and resulting in improvements in the operability of the treatment part 3. Note here that the engaging protrusion 15e and recess 11c define together a stopper.

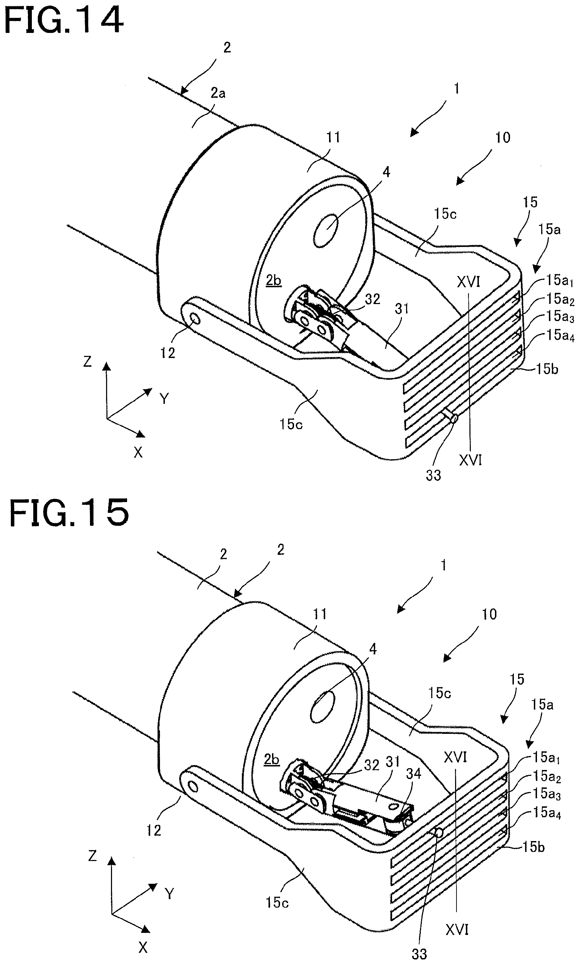

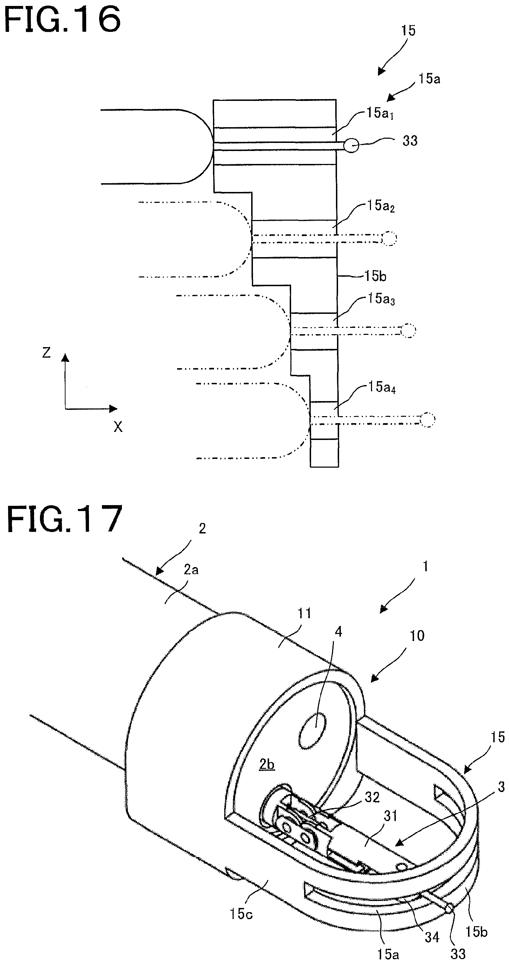

Fig. 14 is a schematic view of one exemplary state where the treatment part 3 turns downward with respect to the cap 10 for medical equipment according to the fourth embodiment, and Fig. 15 is a schematic view of one exemplary state where the treatment part 3 turns upward with respect to the cap 10 for medical equipment according to the fourth embodiment. Fig. 16 is a view of Figs. 14 and 15 as taken on XVI-XVI section. In Figs. 14, 15 and 16 here, a direction of elongation of the side portion 15c of the blade 15 is defined as a first direction X; a direction of connecting the side portions 15c is defined as a second direction Y; and a direction orthogonal to the first X and second direction Y is defined as a third direction Z.

In the cap 10 for medical equipment according to the fourth embodiment, the front portion 15b of the blade 15 is provided with a plurality of surgical openings 15a, as shown in Figs. 14 and 15. In the cap 10 for medical equipment according to the fourth embodiment, more specifically, rectangular openings 15a, each one having a long side in the second direction Y, are arranged in the third direction Z. Note here that the shaft member 12, side portions 15c and front portion 15b define together an extension assembly in the cap 10 for medical equipment according to the fourth embodiment and the side portions 15c define a lateral portion and an arm portion.

It is to be noted that the configuration and direction of arrangement of the surgical openings 15a are not limited to those of the cap 10 for medical equipment according to the fourth embodiment. For instance, rectangular openings 15a longer in the third direction Z may be arranged in the second direction Y, and a plurality of surgical openings 15a may be formed in any desired shape and in any desired positions.

In the cap 10 for medical equipment according to the fourth embodiment, a plurality of surgical openings 15a are provided in the front portion 15b of the blade 15 thereof so that an easy-to-use opening can be selected for insertion of the distal-end portion 33 of the treatment part 3, ensuring that the affected site 100 can be treated with no interference of the treatment part 3 with the cap 10.

In the cap 10 for medical equipment according to the fourth embodiment, the thickness of the front portion 15b is partially varied as shown in Fig. 16 so that the plurality of surgical openings 15a have varying insertion lengths in the first direction X. In the cap 10 for medical equipment according to the fourth embodiment, the front portion 15b has the largest thickness around the uppermost first opening 15a₁ in the third direction; it has the second largest thickness around the second opening 15a₂; it has the third largest thickness around the third opening 15a₃; and the front portion 15b has the smallest thickness around the lowermost fourth opening 15a₄ on the drawing sheet plane. In other words, the first opening 15a₁ has the longest insertion length and the fourth opening 15a₄ has the shortest insertion length.

It is here to be noted that the order of insertion length of the plurality of surgical openings 15a is not limited to that in the direction of the fourth embodiment. For instance, the lowermost opening may have the longest insertion length in the third direction Z in Figs. 14 and 15 whereas the uppermost opening may have the shortest insertion length. When the surgical openings 15a are arranged in the second direction Y, one may have the longest insertion length whereas the other may have the shortest insertion length. Further, it is not always necessary to arrange the insertion openings in descending order from the longest insertion opening 15a to the shortest insertion opening 15a; the longest or shortest insertion opening may be positioned in between. For instance, the second insertion opening 15a₂ or the third insertion opening 15a₃ may have the longest or shortest insertion length.

In the cap 10 for medical equipment according to the fourth embodiment, the insertion lengths of the plurality of surgical openings 15a are partially varied depending on the position of the front portion 15b of the blade 15, as mentioned above. It is thus possible to adjust the length of extension of the distal-end portion 33 of the treatment part 3 out of the surgical opening 15a of the blade 15, thereby avoiding accidental perforation or the like.

Fig. 17 is a schematic view of the cap for medical equipment according to the fifth embodiment.

In the cap 10 for medical equipment according to the fifth embodiment, the base member 11 and blade 15 are integrally formed of a flexible member. When they are provided as separate members, at least the blade 15 in general may have flexibility, and at least the side portions 15c of the blade 15 in particular may be of flexibility. Note here that the side portions 15c and front portion 15b of the cap 10 for medical equipment according to the fifth embodiment define together an extension assembly, and the side portions 15c define a lateral portion and an arm portion.

In the cap 10 for medical equipment according to the fifth embodiment, the actuation of the treatment part 3 inserted into the surgical opening 15a causes the flexible blade 15 to deform in conformity with the movement of the treatment part 3. Thereafter, when the treatment part 3 goes back to the initial position or comes out of the surgical opening 15a in the blade 15, the blade 15 is restored back to its original configuration.

In the cap 10 for medical equipment according to the fifth embodiment, the side portions 15c of the blade 15 are formed of a flexible member, and are just simply driven in harmony with the movement of the treatment part 3, as described above. It is thus possible to treat the affected site 100 with no interference of the treatment part 3 with the cap 10.

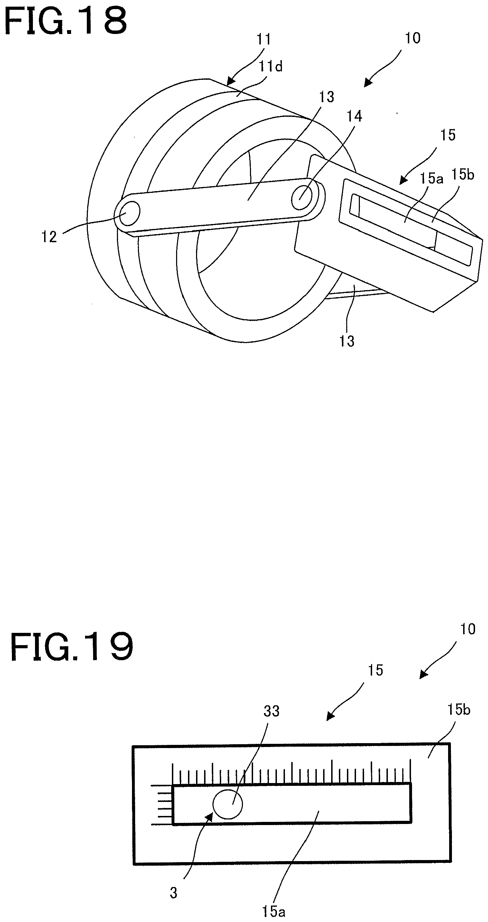

Fig. 18 is a schematic view of the cap for medical equipment according to the sixth embodiment.

In the cap 10 for medical equipment according to the sixth embodiment, the base member 11 is provided in its outer circumference with a groove 11d for making the shaft member 12 of the cap 10 for medical equipment according to the first embodiment shown in Fig. 1 movable. In the cap 10 for medical equipment according to the sixth embodiment, the base member 11 is provided with the groove 11d in the peripheral direction of the outer circumference of the base member 11, but it may be provided in other direction.

In the cap 10 for medical equipment according to the sixth embodiment, the base member 11 is provided in its outer circumference with the groove 11d in which the shaft member 12 is movable. Thus, the treatment part 3 is allowed to have similar functions even when it includes a joint portion capable of axial rotation rather than a combination of bending joints.

Fig. 19 is a schematic view of the cap for medical equipment according to the seventh embodiment.

In the cap 10 for medical equipment according to the seventh embodiment, the front portion 15b of the blade 15 is provided with a scale preferably along the surgical opening 15a in particular. Alternatively, the scale may be provided in such a way as to surround the surgical opening 15a.

In the cap 10 for medical equipment according to the seventh embodiment, the front portion 15b of the blade 15 is provided with the scale as mentioned above. It is thus possible to put the treatment part 3 into operation while checking up with the scale thereby measuring the contour of a tissue and quantitatively cutting open a range of interest.

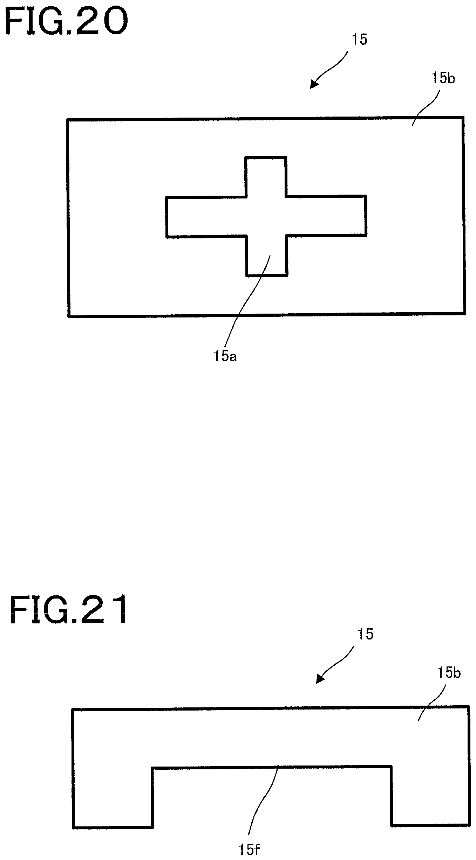

Fig. 20 is illustrative of one example of the surgical opening in the cap for medical equipment according to one embodiment.

Although the surgical opening 15a in the cap 10 for medical equipment according to the embodiment described herein is provided in a rectangular shape, it may have any other desired configuration. For instance, the opening may be configured into a cross shape as shown in Fig. 20 or, alternatively, it may be configured into a circular or oval shape.

Fig. 21 shows one exemplary shape of the front portion of the blade in the cap for medical equipment according to one embodiment.

While the blade 15 in the cap 10 for medical equipment according to the embodiment described herein is provided in the front portion 15b with the surgical opening 15a, it is to be understood that the front portion 15b may have a varying configuration. As shown typically in Fig. 21, it may be provided with a notch 15f that may be configured into any other shape.

In what follows, the surgical system 90 will be explained as an example of the medical system to which the medical equipment 1 incorporating the cap 10 for medical equipment according to one embodiment is applied.

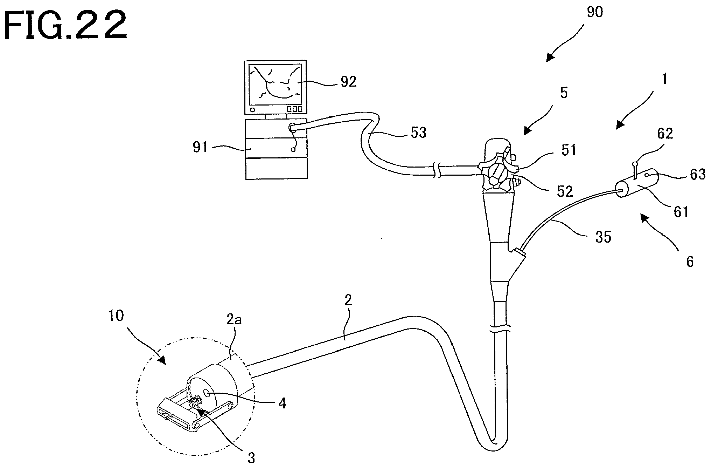

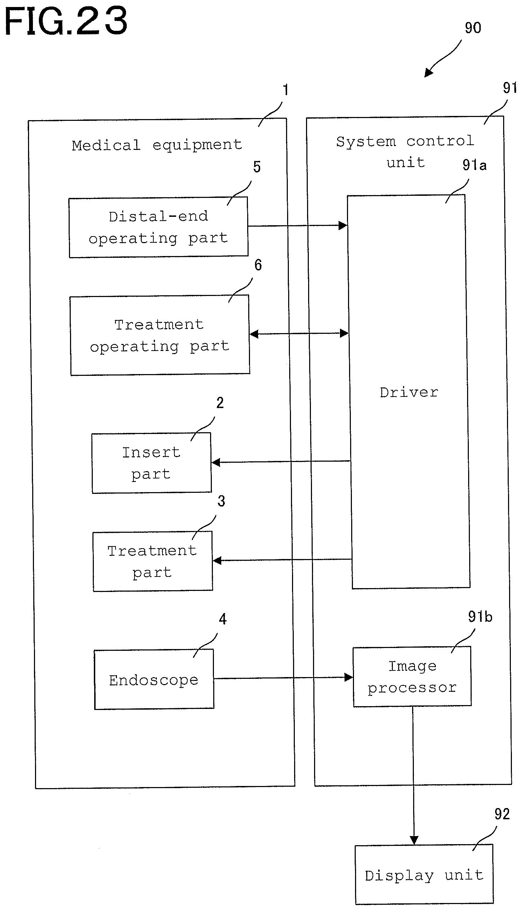

Fig. 22 is illustrative of the surgical system 90 to which the medical equipment 1 incorporating the cap 10 for medical equipment according to one embodiment is applied, and Fig. 23 is illustrative in system architecture of the surgical system 90 to which the medical equipment 1 incorporating the cap 10 for medical equipment according to one embodiment is applied.

The surgical system 90 described herein incorporates the medical equipment 1 incorporating the cap 10 for medical equipment. This surgical system 90 includes the medical equipment 1 having an insert part 2 capable of insertion through the body cavity of a patient, a system control unit 91 for controlling the medical equipment 1, and a display unit 92 for displaying images acquired by a medical apparatus 100.

The distal-end operating part 5 includes a first angle lever 51 for operating the bending of the insert part 2 in a given one direction, and a second angle lever 62 for operating the bending of the insert part 2 in a direction orthogonal to the given one direction of the insert part 2. The direction of the distal-end portion 2a may be varied by the operation of the first 51 and the second angle lever 52.

The treatment operating part 6 includes a driver portion 61 for driving the treatment part 3, a joint operating component 62 for putting the first 32 and second joint portion 34 of the treatment part 3 shown in Fig. 1 in operation, and a switch 63 for applying power to a electric scalpel. By driving the driver portion 61 to put the joint operating component 62 for putting the joints and switch 63 for applying power to the electric scalpel into operation, it is possible to put the treatment part 3 into operation.

In the system control unit 91, the distal-end operating part 5 and treatment operating part 6 are put into operation to actuate the insert part 2 and treatment part 3 via a driver 91a. Images acquired as by the endoscope 4 are produced out to an image processor 91b in the system control unit 91, and images processed by the image processor 91b are displayed on the display unit 92. Then, the operator manipulates the medical equipment 1 while viewing the images displayed on the display unit 92.

According to such a surgical system 90, it is possible to display unerring images asked for by the operator. It is thus possible to improve on the operability of the medical equipment 1 thereby putting the medical equipment 1 into unerring operation.

While the medical equipment 1 used herein includes a flexible distal-end portion 2a, it is to be understood that the distal-end portion 2a may be a hard one, and that the medical equipment 1 may be of the overtube type that includes a passage through the endoscope.

In one embodiment as described above, the cap 10 for medical equipment includes the base member 11 attached to the distal end of the medical equipment 1 and the extension assembly defined by 12, 13, 14 and 15 extending out of the base member 11. The extension assembly defined by 12, 13, 14 and 15 is put into passive operation so that they are able to improve on the operability of the treatment part 3 while acting as a cover for decreasing the deposition of stains.

In one embodiment as described above, the extension assembly defined by 12, 13, 14 and 15 is put into operation as the treatment part 3 used for treatment of the affected site 100 by the medical equipment is put into operation. It is thus possible to direct the extension assembly defined by 12, 13, 14 and 15 as a cover to the affected site 100 in association with the operation of the treatment part 3.

In one embodiment of the cap 10 for medical equipment as described above, the extension assembly defined by 12, 13, 14 and 15 includes two lateral portions 13, 14, 15c supported on the base member 11, and the front portion 15b coupled to the two lateral portions 13, 14 and 15c. Thus, this arrangement permits the lateral portions 13, 14 and 15c to improve on the operability of the treatment part 3 while the front portion 15b acts as a cover for decreasing the deposition of stains.

In one embodiment of the cap 10 for medical equipment as described above, the treatment part 3 is provided with the surgical opening 15a through the treatment part 3 can be inserted. It is thus possible to further decrease the deposition of stains and improve on the operability of the treatment part 3.

In one embodiment of the cap 10 for medical equipment as described above, there are a plurality of surgical openings 15a provided so that depending on a particular state of the affected site, a surgical opening 15a for providing for easy insertion can be chosen for insertion of the treatment part 3, resulting in further improvements in the operability of the treatment part 3.

In one embodiment of the cap 10 for medical equipment as described above, at least one of the surgical openings 15a has an insertion length different from that of other surgical opening 15a. It is thus possible to adjust the length of extension of the surgical distal-end portion 33 of the treatment part 3 out of the surgical opening 15a through the blade 15 depending on a particular state of the affected site 100, resulting in improvements in the operability of the treatment part 3.

In one embodiment of the cap 10 for medical equipment as described above, the front portion 15c is transparent or semitransparent. It is thus possible to make further improvements in the operability of the treatment part 3.

In one embodiment of the cap 10 for medical equipment as described above, the lateral portion 15c includes the arm potion 15c and the shaft member 12 for swingably connecting the arm portion 15c to the base member 11. It is thus possible to set up the cap 10 for medical equipment just simply.

In one embodiment of the cap 10 for medical equipment as described above, the arm portions 13 and 14 include the link member 13 having a proximal end and a distal end, and the coupling member 14 for swingably connecting the base member 11 and shaft member 12 to a shaft at the proximal end and rotatably attaching the front portion 15b at the distal end. It is thus possible to set up the cap 10 for medical equipment just simply.

In one embodiment of the cap 10 for medical equipment as described above, the arm portion 15c can be retained at a plurality of angles relative to the base member 11. It is thus possible to use the treatment part 3 in a stabilized state.

In one embodiment of the cap 10 for medical equipment as described above, the base member 11 is provided with a stopper such that the swinging angle of the arm portion 15c is restricted. It is thus just simply possible to retain the arm portion 15c at a plurality of angles relative to the shaft member 12.

In one embodiment of the cap 10 for medical equipment as described above, the arm portion 15c is movable relative to the shaft member 12. It is thus possible to store the extension component 15 on the side of the distal-end portion 2a of the medical equipment 1 at a time when it is not used thereby improving on the operability of the medical equipment 1.

In one embodiment of the cap 10 for medical equipment as described above, the arm portion 15c includes a slot opening for movably retaining the shaft member 12. It is thus possible to make the shaft member 12 just simply movable.

In one embodiment as described above, the cap 10 for medical equipment includes the treating mode capable of putting the extension assembly into passive operation, and the moving mode of moving and retaining the arm portion 15c of the extension assembly on the base member 11 side relative to the shaft member 12. Upon delivery of the medical equipment 1 to the affected site, therefore, the shaft member 12 is retained in such a way as to prevent the blade 15 from staggering, and from blocking off the field of vision of the medical equipment 1 or disturbing the movement of the medical equipment 1. The treatment part 3 attached to the medical equipment 1 is pushed out of the medical equipment 1 so that the shaft member 12 can move relative to the blade 15 and make a transition to the treating mode.

In one embodiment of the cap 10 for medical equipment as described above, one of the lateral portions 15c is supported on the base member 12, and the other includes the flexible arm portion 15c coupled to the front portion 15b. It is thus possible to improve on the operability of the treatment part 3 just simply while allowing the treatment part to act as a cover for decreasing the deposition of stains.

It is here to be appreciated that the invention is in no sense limited to such embodiments as described above. While the explanation of some embodiments embraces numerous specific details for illustration, it would be obvious to those skilled in the art that diverse variations or modifications made thereto are included within the scope. In other words, illustrative embodiments are described without excluding generality from the claimed inventions and imposing any limitation thereon.

### Reference Signs List

1: Medical equipment
2: Insert part
2a: Distal-end portion
3: Treatment part
4: Endoscope
5: Distal-end operating part
6: Treatment operating part
10: Cap for medical equipment
11: Base member
12: Shaft member (an extension assembly/lateral portion)
13: Link member (an extension assembly, lateral/arm portion)
14: Coupling member (an extension assembly/lateral portion)
15: Blade (an extension assembly)
15a: Surgical opening
15b: Front portion
15c: Side portion (a lateral/arm portion)
90: Medical system

## Claims

1. A cap for medical equipment, **characterized by** comprising:
a base member attached to a distal end of medical equipment; and
an extension assembly that extends out of the base member,
wherein the extension assembly is put into passive operation.

2. A cap for medical equipment according to claim 1,
wherein the extension assembly is put into operation as a treatment part used upon treatment of an affected site by the medical equipment is put into operation.

3. A cap for medical equipment according to claim 1 or 2,
wherein the extension assembly includes two lateral portions supported on the base member, and a front portion coupled to the two lateral portions.

4. A cap for medical equipment according to claim 3,
wherein the front portion is provided with a surgical opening through which the treatment part can be inserted.

5. A cap for medical equipment according to claim 4,
which further includes a plurality of the surgical openings.

6. A cap for medical equipment according to claim 5,
wherein at least one of the surgical openings has an insertion length different from that of the other surgical opening(s).

7. A cap for medical equipment according to any one of claims 3 to 6,
wherein the front portion is transparent or semitransparent.

8. A cap for medical equipment according to any one of claims 3 to 7,
wherein the lateral portions each include an arm portion and a shaft portion for swingably connecting the arm portion to the base member.

9. A cap for medical equipment according to claim 8,
wherein the arm portion includes a link member having a proximal end and a distal end, and a coupling member for swingably connecting the base member and the shaft member to a shaft at the proximal end and for rotatable attachment of the front portion at the distal end.

10. A cap for medical equipment according to claim 8 or 9,
wherein the arm portion can be retained at a plurality of angles relative to the base member.

11. A cap for medical equipment according to claim 8 or 9,
wherein the base member is provided with a stopper in such a way as to restrict an angle of swinging of the arm member.

12. A cap for medical equipment according to any one of claims 8 to 11,
wherein the arm portion is capable of movement relative to the shaft member.

13. A cap for medical equipment according to claim 12,
wherein the arm portion includes an opening formed as a slot for movably supporting the shaft member.

14. A cap for medical equipment according to claim 12 or 13,
which includes a treating mode capable of putting the extension assembly into passive operation, and a moving mode of moving the arm portion of the extension assembly on a base member side relative to the shaft member and retaining there.

15. A cap for medical equipment according to any one of claims 3 to 7,
wherein the lateral portion includes flexible arms one of which is supported on the base member and the other of which is coupled to the front portion.
